# EUROPÄISCHE PATENTANMELDUNG

(11) **EP 1 125 598 A2**
(43) Veröffentlichungstag der Anmeldung: **22.08.2001**
(21) Anmeldenummer: 01250053.4
(22) Anmeldetag: 19.02.2001
(51) Int. Cl.: A61N 1/365

(54) **Medizinisches Therapiegerät und Sensor zum Erlangen von molekulargenetischer Information für das Gerät**

(30) Priorität: 19.02.2000 DE 10007715
(71) Anmelder: BIOTRONIK Mess- und Therapiegeräte GmbH & Co Ingenieurbüro Berlin, 12359 Berlin (DE)
(72) Erfinder: Schaldach, Max, Prof. Dr., 91054 Erlangen (DE)
(74) Vertreter: Eisenführ, Speiser & Partner

(57) **Zusammenfassung**

Die Erfindung betrifft einen Sensor zum Erfassen von Information über den Zustand eines Organismus für ein medizinisches Therapiegerät, vorzugsweise für ein Therapiegerät zur Elektrostimulation oder anderweitigen Behandlung des Herzens. Die Erfindung zeichnet sich durch mindestens ein Sensorelement zum Erfassen von molekulargenetischer Information aus. Die Erfindung betrifft weiterhin ein medizinisches Therapiegerät mit einem derartigen Sensor.

## Beschreibung

Die Erfindung betrifft einen Sensor zum Erfassen von Information über den Zustand eines Organismus für ein medizinisches Therapiegerät, vorzugsweise für ein Therapiegerät zur Elektrostimulation oder anderweitigen Behandlung des Herzens.

Derartige Sensoren sind aus dem Stand der Technik bekannt. Die von ihnen erfaßten Informationen werden von den medizinischen Therapiegeräten benötigt, um optimal arbeiten zu können. So erfassen beispielsweise Sensoren für Therapiegeräte zur Elektrostimulation des Herzens, insbesondere Sensoren für Herzschrittmacher oderfür Defibrillatoren, Herzrhythmusstörungen. Sensoren elektromedizinischer Geräte können jedoch auch Muskelaktivität, Lungenfunktionsparameter, die Sauerstoffsättigung, den Blutdruck, den Hormonspiegel oder andere physiologische Parameter messen. All diese Parameter können zur Steuerung des Herzschrittmachers oder des Defibrillators verwendet werden.

Insbesondere bei Herzschrittmachern ist es dabei wichtig, den von dem Herzschrittmachererzeugten Stimulationsimpuls bezüglich seiner Stimulationsamplitude an die Stimulationsreizschwelle anzupassen. Die Stimulationsreizschwelle kennzeichnet diejenige Stimulationsamplitude des Herzschrittmachers, die erforderlich ist, um einen Stimulationserfolg, d.h. eine stimulierte Systole auszulösen. Da sich die Stimulationsreizschwelle infolge des Hormonspiegels, der Tageszeit, der körperlichen Aktivität usw. ändern kann, ist es vorteilhaft, wenn die Stimulationsamplitude dieser Änderung der Stimulationsreizschwelle anpaßbar ist. Zur Steuerung des Herzschrittmachers kann dabei auch der Stimulationserfolg selbst verwendet werden. Denn wird beispielsweise durch einen ausbleibenden Stimulationserfolg nach Abgabe eines Stimulationsimpulses (infolge eines ausbleibenden Impedanzanstieges im Impedanzverlauf nach Abgabe des Stimulationsimpulses) eine unterschwellige Stimulation von einem entsprechenden Sensor erkannt, so ist die Stimulationsamplitude zu erhöhen, um weiterhin eine zuverlässige Funktion des Herzschrittmachers sicherzustellen. Zusammenfassend läßt sich festhalten, daß bei elektromedizinischen Therapiegeräten, insbesondere bei Herzschrittmachern und Defibrillatoren, eine Optimierung der Funktion des Gerätes umso stärker möglich ist, je mehr Parameter in die Steuerung bzw. Regelung des Gerätes eingehen.

Aufgabe der vorliegenden Erfindung ist es daher, einen Sensor der eingangs genannten Art derart weiterzuentwickeln, daß zusätzliche, bisher im Stand der Technik nicht erfaßte Parameter erfaßbar sind.

Diese Aufgabe wird bei einem Sensor der eingangs genannten Art dadurch gelöst, daß mindestens ein Sensorelement zum Erfassen von molekulargenetischer Information vorgesehen ist. Darüber hinaus stellt die Erfindung ein medizinisches Therapiegerät, insbesondere einen Herzschrittmacher oder einen Defibrillator zur Verfügung, welches einen derartigen Sensor aufweist.

Die Vorteile der Erfindung liegen insbesondere darin, daß aufgrund der Erfassung molekulargenetischer Information ein weiterer Parameter zur Verfügung steht, um ein medizinisches Therapiegerät zu steuern. Ein derartiger Sensor, der wie das Therapiegerät selbst innerhalb oder außerhalb des Körpers angebracht sein kann, kann somit vorteilhafterweise beispielsweise genetische Defekte erfassen, die eine Änderung der Stimulationsreizschwelle bei Herzschrittmachern zur Folge haben. Auf diese Weise kann ein so ausgestattetes medizinisches Therapiegerät die Stimulationsreizschwelle auch an derartige dauerhaft vorhandene Eigenarten des jeweiligen Organismus anpassen. Die erfindungsgemäßen Sensoren können modulartig Teil eines Therpiegerätes sein, so daß sie jederzeit austauschbar oder auch nachträglich ergänzbar sind. Die erfindungsgemäßen Sensoren können jedoch auch fester integraler Bestandteil eines medizinischen Therapiegerätes sein.

Darüber hinaus ist es dank der Erfindung vorteilhaft möglich, neben klinischen Indikatoren für Herzrhythmusstörungen oder anderen Fehlfunktionen des Organismus auch genetische bzw. biologische Indikatoren zu bestimmen. Auf diese Weise kann dank der Erfindung beispielsweise eine besondere Anfälligkeit des Organismus für Herzrhythmusstörungen oder auch für bestimmte Arten von Herzrhythmusstörungen frühzeitig erfaßt bzw, bestimmt werden. Ist ein solcher erfindungsgemäßer Sensor dann Teil eines medizinischen Therapiegerätes, so kann unter Einbeziehung eines solchen genetischen Parameters ein Indikatorsignal als Maß für ein bevorstehendes therapiebedürftiges Ereignis bestimmt werden. Bestimmte behandlungsbedürftige physiologische Zustände des Organismus können somit schon im Vorwege erkannt und somit der Einsatz entsprechender Therapiemaßnahmen des Therapiegerätes initiiert oder vorbereitet werden.

Bei einer vorteilhaften Ausführungsform der Erfindung weist das Sensorelement mindestens ein Andockelement auf, an welchem Andockelement Moleküle des Organismus andocken können, wobei die Andockspezifizität des Andockelementes bekannt ist. Mit Hilfe eines solchen Andockelementes kann somit festgestellt werden, ob bestimmte Moleküle in dem Organismus vorhanden sind. Wird dabei die Andockspezifizität des Andockelementes beispielsweise so eingestellt, daß bestimmte genetische Defekte, die sich in bestimmten spezial ausgebildeten Molekülen im Organismus äußern, auf diese Weise festgestellt werden können, so kann der Sensor diesen genetischen Effekt feststellen und ggf. an ein Therapiegerät weitermelden. Das Therapiegerät kann dann seinerseits wiederum - ggf. nach Einstellung oder Freigabe durch den Arzt - eine entsprechende Therapiemaßnahme einleiten oder die Stärke der Therapiemaßnahme dem festgestellten genetischen Defekt anpassen. Bei Herzschrittmachern kann in diesem Zusammenhang die Stimulationsreizschwelle entsprechend den bekannten Auswirkungen des festgestellten genetischen Defektes angehoben oder abgesenkt werden.

Um die oben genannten bekannten Moleküle festzustellen, kann das Andockelement selbst ein bekanntes Molekül enthalten. Bei diesen bekannten Molekülen handelt es sich vorzugsweise um synthetische Oligonukleotide oder um PCR-generierte cDNA-Fragmente. Mit Hilfe derartiger Oligonukleotide oder cDNA lassen sich komplementäre DNA-Stücke des zu untersuchenden Organismus binden. Da die als Andockelemente dienenden Oligonukleotide oder cDNA-Fragmente bekannt sind, ist somit auch das gebundene DNA-Stück bekannt. Aus dem Vorhandensein eines bestimmten DNA-Stückes kann dann im Rückschluß auf einen bestimmten Zustand des Organismus oder auf eine krankhafte Veränderung des Organismus geschlossen werden. Auf diese Weise kann beispielsweise auch das Vorhandensein oder die Menge bestimmter Enzyme im Organismus festgestellt werden. Dies kann bei der Früherkennung eines Herzinfarktes hilfreich sein.

Bei einer weiteren bevorzugten Ausführungsform sind die Sensorelemente bevorzugt mindestens zweifach auf dem Sensor vorhanden. Somit können zufällige Bindungen von Molekülen an den Andockelementen als zufällig erkannt und für die Auswertung des Meßergebnisses ausgeschlossen werden.

Besonders bevorzugt enthält der erfindungsgemäße Sensor Meßelemente, die bevorzugt mit jedem Sensorelement verbunden sind, und welche Meßelemente die Hybridisierung eines komplementären Moleküls des Organismus an das als Andockelement dienende bekannte Molekül erfassen. Bei den Meßelementen kann es sich dabei um ein Strommeßelement zum Messen eines durch die Hybridisierung erzeugten elektrischen Stromes handeln, um ein Fluoreszenzmeßelement zum Erfassen einer durch die Hybridisierung vorhandenen Fluoreszenz, um ein Ladungsmeßelement zum Erfassen einer durch die Hybridisierung geänderten elektrischen Ladungsverteilung handeln, oder um ein Strahlungsmeßelement zum Erfassen einer durch die Hybridisierung vorhandenen radioaktiven Strahlung handeln. Handelt es sich beispielsweise um ein Meßelement zur Erfassung einer Fluoreszenz, so wird die zu untersuchende DNA oder RNA des Organismus entsprechend mit Fluoreszenzmarkierungen versehen. Hybridisiert eine derartig markierte DNA oder RNA mit den komplementären Sequenzen auf dem Sensor, so läßt sich diese Fluoreszenz und somit die Hybridisierung mit dem Fluoreszenzmeßelement erfassen.

Bei einer besonders bevorzugten Ausführungsform der vorliegenden Erfindung sind die als Andockelemente dienenden bekannten Moleküle schachbrettartig in mehreren Reihen nebeneinander angeordnet. Auf diesem schachbrettartig mit den Sensorelementen versehenen Sensor werden dann für einen bestimmten genetischen Defekt oder einen bestimmten molekulargenetischen Zustand charakteristische Oligonukleotide oder cDNA-Fragmente in einer bestimmten Reihenfolge bzw. einem bestimmten Muster angeordnet. Für dieses Muster ist es wiederum bekannt, in welcher Weise bei einem bekannten genetischen Defekt die DNA oder RNA des Organismus, welcher diesen genetischen Defekt aufweist, an die schachbrettartig angeordneten Moleküle bindet. Es ist somit für ein bestimmtes Gen oder einen bestimmten genetischen Defekt ein bestimmtes Bindungs- oder Hybridisierungsmuster bekannt. Dieses Hybridisierungsmuster kann beispielsweise in einem Speicher des Sensors gespeichert sein. Wird der Sensor dann zur Untersuchung eines Organismus eingesetzt, so ist mit Hilfe der Meßelemente das dann tatsächlich vorhandene Muster erfaßbar und mit Hilfe von einer Vergleichsvorrichtung mit den gespeicherten Hybridisierungsmustern vergleichbar. Steht mit Hilfe des Vergleichsmittels dann eine Übereinstimmung des aktuellen Hybridisierungsmusters mit einem gespeicherten Hybridisierungsmuster fest, so ist der Sensor damit in der Lage, das Vorhandensein eines bestimmten genetischen Defektes oder auch eines bestimmten molekulargenetischen Zustandes des untersuchten Organismus festzustellen. Dabei ist es auch möglich, den zeitlichen Ablauf des Hybridisierungsmusters festzustellen. Auf diese Weise läßt sich beispielsweise die Expression bestimmter Gene nachvollziehen. Aus einer solchen gemessenen Expression von bestimmten Genen lassen sich wiederum Rückschlüsse auf einen bestimmten Zustand des Organismus ziehen. Wird beispielsweise mit Hilfe des erfindungsgemäßen Sensors festgestellt, daß in dem Organismus bestimmte Gene exprimiert werden, die nur in einem bestimmten krankhaft veränderten Zustand des Organismus exprimiert werden, so kann der Sensor ein entsprechendes Signal erzeugen, welches beispielsweise einem medizinischen Therapiegerät den genetischen Defekt des Organismus oder die krankhaft veränderte Expression bestimmter Gene meldet. Auch die krankhaft überhöhte Produktion bestimmter Enzyme läßt sich somit erfassen.

Die oben erwähnten bekannten Moleküle sind bevorzugt auf einem Trägersubstrat aus Glas, Gel beschichtetem Glas, Nylon, Silikat, Silizium oder Galliumarsenid angeordnet. Mit dem Trägersubstrat verbunden sind dann bevorzugt entsprechende Meßelemente. Dabei ist es erfindungsgemäß besonders bevorzugt, wenn es sich bei den Meßelementen um Meßelemente zum Messen eines elektrischen Stromes handelt. Auf diese Weise läßt sich die erfinderische Idee besonders einfach verwirklichen, indem an jedem Sensorelement ein elektrischer Strom oder eine Änderung des elektrischen Stromes gemessen wird, wenn eine Hybridisierung mit einem Molekül stattgefunden hat.

Bei einer weiteren bevorzugten Ausführungsform werden auf dem schachbrettartigen Sensor cDNA-Fragmente angeordnet, die charakteristisch für die Expression von Genen für bestimmte Hormone sind. Auf diese Weise läßt sich mit Hilfe des erfindungsgemäßen Sensors auch der Hormonhaushalt des überwachten Organismus feststellen, insbesondere das Vorhandensein oder der Überschuß eines bestimmten Hormons festhalten. Insbesondere wenn es sich bei den medizinischen Therapiegeräten der Erfindung um Herzschrittmacher oder Defibrillatoren handelt, ist es von großer Wichtigkeit, bestimmte Hormone im Körper frühzeitig nachweisen zu können. Denn insbesondere die Stimulationsreizschwelle für einen Herzschrittmacher ist u.a. abhängig von einem bestimmten Hormonspiegel im Organismus. Durch molekulargenetische Feststellung des Hormonspiegels mit Hilfe des erfindungsgemäßen Sensors läßt sich dann bei einem solchen mit dem erfindungsgemäßen Sensor ausgerüsteten Herzschrittmacher der Herzschrittmacher in seiner Funktion optimiert einsetzen.

Weiterhin ist es vorteilhaft, wenn das erfindungsgemäße Sensorelement medizinisches Therapiegerät, insbesondere medizinisches Therapiegerät zur Elektrostimulation oder anderweitigen Behandlung des Herzens mit einem eingangsseitigen Sensormittel zum Erzeugen mindestens eines Eingangssignals versehen ist, welches als Indikatorsignal ein Maß für eine Therapieschwelle oder eine notwendige Therapieintensität bildet. Auf diese Weise kann die Therapieschwelle oder -intensität selbsttätig auf die genetische Prädisposition des Patienten eingestellt werden.

Insbesondere günstig ist es, wenn bei einem implantierbar ausgebildeten elektromedizinischen Gerät das Indikatorsignal, mindestens indirekt ein Eingangssignal für ein Programmiergerät bildet, welches mindestens mittelbar zur Einstellung von mindestens einem Therapie- oder Betriebszustandsparameter des Therapiegeräts dient. Auf diese Weise kann das Therapiegerät bereits bei der Herstellung oder unmittelbar nach der Implantation individuell für einen bestimmten Patienten (vor-) eingestellt werden. Ein entsprechendes Herstellungsverfahren für ein medizinisches Therapiegerät ist hierbei ebenfalls Gegenstand der Erfindung.

Bei einer bevorzugten Ausführung eines derartigen medizinischen Therapiegerät ist mittels des mindestens einen Indikatorsignals ein Adressiersignal für einen Speicher erzeugbar, der nach Art einer adressierbaren Tabelle (Look-up-table) jeweils in verschiedenen von dem Adressiersignal in Abhängigkeit von dem Indikatorsignal auswählbaren Speicherbereichen alsAusgangssignale auslesbare Therapieinformationen enthält, welche dem jeweiligen Indikatorsignal zugeordnet sind.

Weitere bevorzugte Ausführungsformen der Erfindung sind in den Unteransprüchen angegeben.

Eine bevorzugte Ausführungsform der Erfindung wird nun Bezug auf die Zeichnung beschrieben. Die Figuren der Zeichnung zeigen:
- Fig. 1: einen erfindungsgemäßen Sensor in einer schematischen Aufsicht und einer schematischen Seitenansicht;
- Fig. 2: den Sensor der Fig. 1 nach Bindung von Molekülen an den Sensor;
- Fig. 3: ein Ausführungsbeispiel in prinzipieller Blockdarstellung, bei dem die Ausgangssignale eineserfindungsgemäßen Sensors zur Programmierung eines Therapiegeräts herangezogen werden; und
- Fig. 4: eine Detaildarstellung zu verschiedenen Blöcken der Darstellung nach Fig. 3.

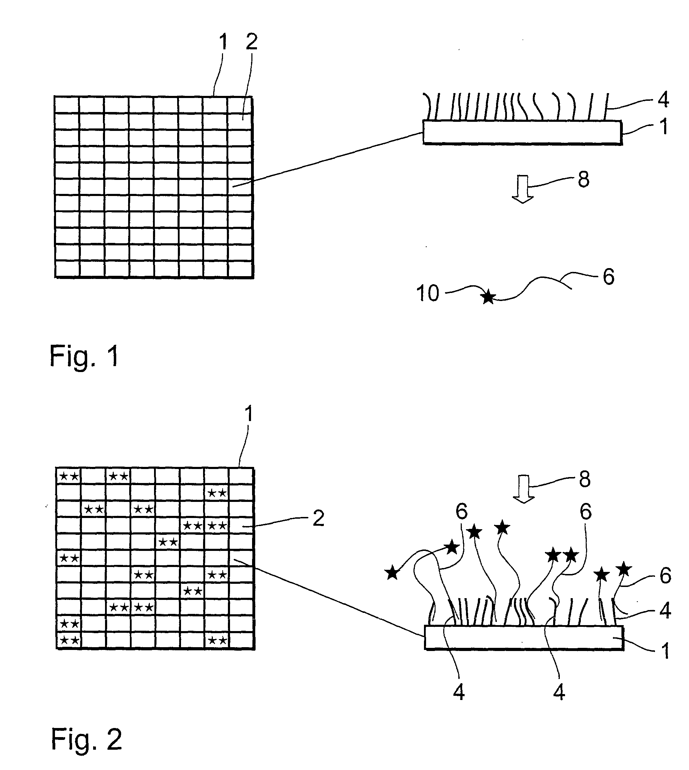

Fig. 1 zeigt in dem in der Fig. links dargestellten Teil einen Sensor 1 in einer Aufsicht. Der Sensor 1 weist einen im wesentlichen quadratischen Grundriß auf und trägt schachbrettartig auf dieser quadratischen Fläche angeordnet 88 Sensorelemente 2.

In dem in der Zeichnung rechts dargestellten Teil der Fig. 1 ist der Sensor 1 in einer Seitenansicht dargestellt. Dabei wird eine Reihe von Sensorelementen 2 schematisch in der Seitenansicht deutlich. Jedes der Sensorelemente 2 trägt zwei als erfindungsgemäße Andockelemente dienende cDNA-Fragmente 4. Diese cDNA-Fragmente 4 sind jeweils paarweise identisch. Die Sequenz jedes cDNA-Fragmentes ist spezifisch für ein bestimmtes ausgewähltes Gen.

Wird der Sensor 1 nun mit aus Zellen oder Gewebe des zu untersuchenden Organismus isolierter DNA oder RNA 6 wie durch die Pfeile 8 symbolisch angedeutet in Kontakt gebracht, so binden nur bestimmte Abschnitte der isolierten DNA oder RNA an bestimmte cDNA-Fragmente 4 auf dem Sensor 1. Diese Bindung der DNA oder RNA 6 an die cDNA-Fragmente 4 ist in dem in der Fig. rechts dargestellten Teil der Fig. 2 dargestellt. Durch das Hybridisieren der komplementären Sequenzen der isolierten DNA oder RNA 6 des Organismus an die bekannten cDNA-Fragmente 4 der einzelnen Sensorelemente 2 entsteht auf dem Sensor 1 ein charakteristisches Muster der Hybridisierung. Dieses Hybridisierungsmuster ist in dem in der Zeichnung links dargestellten Teil der Fig. 2 schematisch dargestellt. Das Hybridisierungsmuster wird sichtbar, indem die zu untersuchende DNA oder RNA 6 zuvor mit einem Fluoreszenzmarker 10 markiert wurde. Der Fluoreszenzmarker 10 ist in den Fig. als Sternchen am Ende der isolierten DNA bzw. RNA 6 dargestellt. Die Fluoreszenz der Fluoreszenzmarker 10 läßt sich mit Hilfe geeigneter, als erfindungsgemäße Meßelemente dienender (nicht dargestellter) Scanner erfassen. Somit läßt sich das Hybridisierungsmuster im linken Teil der Fig. 2 aufgrund der Fluoreszenzmarker 10 erfassen.

Das so erfaßte Hybridisierungsmuster kann anschließend mit einem in einem Speicher gespeicherten bekannten Vorrat an Hybridisierungsmustern abgeglichen werden. Wird dann eine Übereinstimmung mit einem in dem Speicher gespeicherten Hybridisierungsmuster festgestellt, so kann der Sensor 1 das Vorhandensein oder die Expression eines bestimmten Gens in dem untersuchten Organismus feststellen und ggf. an ein mit ihm verbundenes Therapiegerät melden. Die Erfassung der für ein bestimmtes Gen, Hormon oder Enzym charakteristischen Hybridisierungsmusters kann bei einem medizinischen Therapiegerät automatisch bewerkstelligt werden, indem von dem Sensor 1 automatisch eine DNA oder RNA 6 des Organismus isoliert wird und automatisch mit den cDNA-Fragmenten 4 in Kontakt gebracht wird, wobei abschließend mit Hilfe der Meßelemente die Hybridisierung der DNA bzw. RNA 6 an bestimmte cDNA-Fragmente 4 automatisch erfaßt wird.

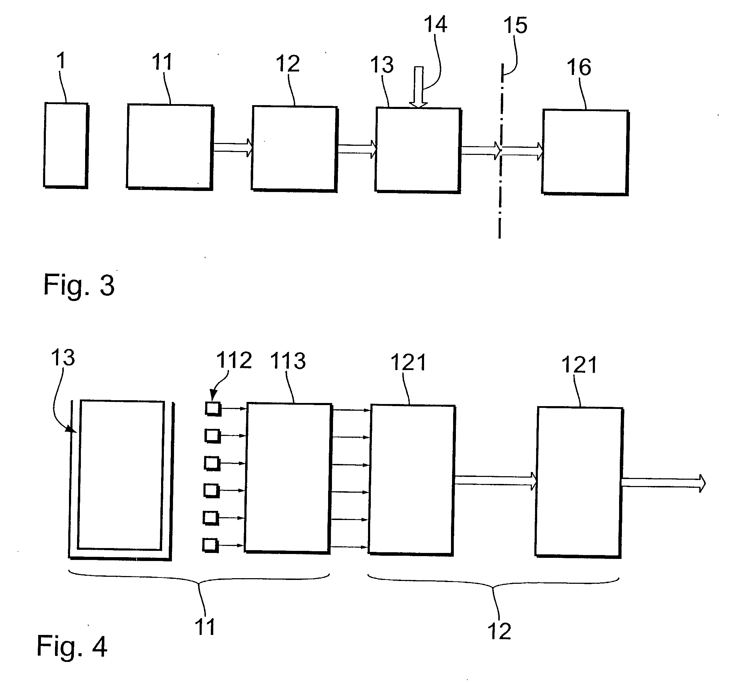

In den Figuren 3 und 4 ist ein Ausführungsbeispiel der Erfindung mit einer Auswertungseinheit für das Sensorelement zusammen mit einem programmierbaren Therapiegerät in Form eines implantierbaren Herzstimulations- und/oder Defibrillationsgeräts jeweils als Blockschaltbild wiedergegeben.

In der Darstellung gemäß Fig. 3 ist eine Messeinheit 11 vorgesehen, welche mindestens ein Messelement zur Auswertung eines Sensorelements aufweist. Das mit einer genetischen Probe des Patienten in Kontakt gebrachte Sensorelement 1 wird in der Messeinheit ausgewertet, in dem - wie zuvor beschrieben - ein die genetische Information beinhaltendes Ausgangssignalmuster des betreffenden Sensorelements abgefragt und einer Auswertungseinheit 12 zugeführt wird. In der Auswertungseinheit 12 wird das Ausgangssignalmuster des Sensorelements in ein Eingangssignal für einen Speicher umgeformt, der - in entsprechend verschlüsselter Form - verschiedene Therapieapieanweisungen in Abhängigkeit von vorgegebenen genetischen Prädispositionen enthält. Hierbei handelt es sich bei dem dargestellten Gerät um Anweisungen, welche die Elektrotherapie des Patienten betreffen. In Abhängigkeit von dem jeweiligen Ausgangssignalmuster wird damit ein Steuersignal für ein Programmiergerät 13 erzeugt, welches das implantierbare Stimulations- bzw. Defibrillationsgerät 15 zusammen mit weiteren vom Arzt unmittelbar eingegebenen Programmier-Steuersignalen 14 - beeinflusst, wenn es in eine Kommunikationsverbindung mit dem Therapiegerät gebracht wird.

Während also bisher die Einstellung eines medizinischen Therapiegeräts ganz von der medizinischen Diagnose durch den Arzt abhing, können die zu programmierenden Parameter jetzt - mindestens zusätzlich - unmittelbar durch die genetische Prädisposition des Patienten beeinflusst werden. So kann beispielsweise die Therapieschwelle oder die notwendige Therapieintensität für jeden Patienten von erblichen Eigenschaften abhängig sein, welche für den betreffenden Patienten eine Therapiekonstante bildet. Diese Therapiekonstante wird einmalig durch die beschriebene genetischen Untersuchung vom Patienten abgeleitet und in die Programmierung der Elektrotherapie des Patienten übernommen, so dass die Behandlung des Patienten besser an dessen individuelle Bedürfnisse angepasst ist.

In Figur 4 sind die Messeinheit 11 und die Auswerteeinheit 12 noch einmal im Detail dargestellt. In den Aufnahmeteil 111 der Messeinheit 11 wird das Sensorelement 1 eingeschoben, welches zuvor mit genetischem Material des Patienten in Reaktionskontakt gebracht worden ist. Der Innenteil der Messeinheit 11 ist mit Messelementen 112 versehen, welche Signale aufnehmen, welche von dem Sensorelement 1, gegebenenfalls unter entsprechenden Aktivierungsbedingungen - abgegeben werden. Bei diesen Aktivierungsbedingungen handelt es ich beispielsweise um Licht 113, wenn die Sensorelemente fluoreszierende Eigenschaften aufweisen oder um einen chemischen Aktivator, wenn die relevanten Signale durch einen chemooptischen Prozess erzeugt werden. Die Ausgangssignale der Messelemente gelangen in jeweils zugeordnete - nicht dargestellte - Zwischenspeicher, welche die jeweiligen Signalzustände zur weiteren Auswertung festhalten, auch wenn der das betreffende Signal auslösende Vorgang bereits abgeschlossen ist. Der Speicherung voran geht ein Digitalisierungsschritt, welcher im einfachsten Fall (einstufig) durch einen Schmitt-Trigger oder aber (mehrstufig) durch eine Analog-Digital-Converter-Einheit bewirkt wird.

Die digitale Ausgangssignale der Messelemente gelangen als Indikatorsignale über ein Interface 114 in einen adressierbaren Speicher 121 der nachgeschalteten Auswerteeinheit 122, wo sie zur weiteren Verarbeitung festgehalten werden. Die Ausgangssignale bilden in digitalisierter Form ein Signalmuster, welches zurAdressierung einer des adressierbaren Speichers in der Form herangezogen wird, dass dieser Speicher eine Nachschlagetabelle (lookup table) bildet, welche für jedes Eingangssignal oder jede relevante Kombination von Eingangssignalen ein Ausgangssignal abgibt, welches eine Therapieeinstellung oder ein Hilfssignal für eine Therapieeinstellung für das zu programmierende Therapiegerät kennzeichnet.

Hierbei handelt es sich beispielsweise um ein Maßsignal für eine für den betreffenden Patienten charakteristische Therapieschwelle oder eine charakteristische Therapieintensität entsprechend der genetischen Prädisposition des Patienten.

Über eine nachgeschaltete Ausgabeeinheit 12 werden die betreffenden Ausgangssignale dem (in Fig. 3 als Block dargestellten) Programmiergerät zugeführt, wo sie allein oder in Kombination mit einem vom Arzt eingestellten (Pfeil 14) Therapieparameter in Steuersignal zur Beeinflussung des implantierbaren Therapiegeräts bilden. Da die genetische Prädisposition des Patienten als im wesentlichen unveränderlich anzusehen ist, brauchen die dargestellten Maßnahmen für jeden Patienten - bevorzugt bereits vor oder unmittelbar nach der Implantation des medizinischen Geräts - nur einmalig durchgeführt zu werden.

Im Falle der Anwendung unmittelbar bei der Herstellung ist es nicht notwendig, dass das Programmiergerät eine getrennte Einheit bildet. Hier wird günstigerweise die Eingabe der entsprechenden Therapieparameter, welche aus dem mindestens einen Indikatorsignal hergeleitet wurde, unmittelbar zu Erzeugung eines programmierbaren Speichers (EPROM etc.), welcher die Therapieparameter des zu erzeugenden Geräts enthält verwendet. Dies kann wohlgemerkt in einem Herstellungsschritt noch vor der Zusammenfügung oder Konfiguration des betreffen medizinischen Geräts erfolgen, so dass bei einer bedarfsgesteuerten Fertigung Geräte erzeugt werden können, welche individuell auf die Bedürfnisse einzelner Patienten abgestimmt sind. Dabei brauchen die Indikatordaten des Sensors nicht unmittelbar in der Fertigungsstätte des Geräts ermittelt zu werden, sondern können beim Patienten erfasst und durch geeignete Kommunikationsmittel zur Fertigungsstätte übertragen werden. Auf diese Weise braucht das Gerät später nicht mehr verändert zu werden.

## Patentansprüche

1. Sensor zum Erfassen von Information über den Zustand eines Organismus für ein medizinisches Therapiegerät, vorzugsweise für ein Therapiegerät zur Elektrostimulation oder anderweitigen Behandlung des Herzens,
gekennzeichnet durch mindestens ein Sensorelement (2) zum Erfassen von molekulargenetischer Information.

2. Sensor nach Anspruch 1,
wobei das Sensorelement (2) mindestens ein Andockelement (4) aufweist, an welches Andockelement (4) Moleküle (6) des Organismus andocken können, wobei die Andockspezifizität des Andockelementes (4) bekannt ist.

3. Sensor nach Anspruch 2,
wobei das Andockelement ein bekanntes Molekül (4) enthält.

4. Sensor nach Anspruch 3,
wobei es sich bei den bekannten Molekülen um synthetische Oligonukleotide und/oder PCR-generierte cDNA-Fragmente (4) handelt.

5. Sensor nach einem der vorstehenden Ansprüche,
wobei mindestens eines der Sensorelemente (2) in identischer Form mindestens zweifach auf dem Sensor (1) vorhanden ist.

6. Sensor nach einem der vorstehenden Ansprüche,
wobei mit der überwiegenden Vielzahl der Sensorelemente (2) mindestens ein Meßelement verbunden ist, welches die Hybridisierung eines komplementären Moleküls (6) des Organismus an das bekannte Molekül (4) erfaßt.

7. Sensor nach Anspruch 6,
wobei das Meßelement ein Strommeßelement zum Messen eines durch die Hybridisierung erzeugten elektrischen Stromes ist, ein Fluoreszenzmeßelement zum Erfassen einer durch die Hybridisierung vorhandenen Fluorreszenz ist, ein Ladungsmeßelement zum Erfassen einer durch die Hybridisierung geänderten elektrischen Ladungsverteilung ist, oder ein Strahlungsmeßelement zum Erfassen einer durch die Hybridisierung vorhandenen radioaktiven Strahlung ist.

8. Sensor nach Anspruch 7,
mit einem Speicher zum Speichern von Hybridisierungszuordnungen für die bekannten Moleküle (4),
wobei mit den Meßelementen ein Vergleichsmittel zum Abgleichen der erfaßten Hybridisierung mit einer in dem Speicher gespeicherten Vorgabe verbunden ist.

9. Sensor nach Anspruch 8,
mit einem Speicher zum Speichern des zeitlichen Verlaufes der Hybridisierung an bekannte Moleküle (4), wobei die Vergleichsmittel zum Abgleichen eines mit Hilfe der Meßelemente erfaßten zeitlichen Verlaufs der Hybridisierung mit dem gespeicherten Verlauf ausgebildet sind.

10. Sensor nach einem der Ansprüche 7 bis 9,
mit einem Speicher zum Speichern von Hybridisierungsmustern der Hybridisierungen an bekannte Moleküle (4),
wobei die Vergleichsmittel zum Abgleichen eines mit Hilfe der Meßelemente erfaßten Hybridisierungsmusters mit einem gespeicherten Hybridisierungsmuster ausgebildet sind.

11. Sensor nach einem der vorstehenden Ansprüche,
wobei die Sensorelemente (2) in einer Reihe angeordnet sind.

12. Sensor nach Anspruch 11,
wobei mehrere etwa gleich lange Reihen nebeneinander schachbrettartig angeordnet sind.

13. Sensor nach einem der vorstehenden Ansprüche,
mit einem Trägersubstrat für die Sensorelemente (2), wobei die Sensorelemente (2) auf dem Trägersubstrat immobilisiert sind.

14. Sensor nach Anspruch 13,
wobei das Trägersubstrat aus Glas, gelbeschichtetem Glas, Nylon, Silikat, Silizium oder Galliumarsenid besteht.

15. Sensor nach einem der vorstehenden Ansprüche,
wobei mindestens 100, bevorzugt mindestens 1.000, weiter bevorzugt mindestens 10.000 verschiedene bevorzugt als Andockelemente (4) ausgebildete Sensorelemente (2) vorgesehen sind.

16. Medizinisches Therapiegerät, insbesondere ein medizinisches Therapiegerät zur Elektrostimulation oder anderweitigen Behandlung des Herzens,
mit mindestens einem zwischen mindestens einem eingangsseitigem Sensormittel und mindestens einem ausgangsseitigen Therapie-Applikationsmittel eingeschalteten Verknüpfungs- und/oder Bearbeitungsmittel,
dadurch gekennzeichnet, daß das Sensormittel einen Sensor (1) nach einem der Ansprüche 1 bis 15 aufweist.

17. Medizinisches Therapiegerät, insbesondere ein medizinisches Therapiegerät zur Elektrostimulation oder anderweitigen Behandlung des Herzens,
mit mindestens einem eingangsseitigen Sensormittel zum Erzeugen mindestens eines Eingangssignals,
mit mindestens einer Verknüpfungs- und/oder Bearbeitungsstufe zum Verarbeiten des Eingangssignals zu einem Indikatorsignal, welches Indikatorsignal ein Maß für ein wahrscheinlich bevorstehendes therapiebedürftiges Ereignis oder einen Therapieerfolg bildet,
mit mindestens einem ausgangsseitigen, von dem Indikatorsignal gesteuerten Therapie-Applikationsmittel, wobei das Eingangssignal und/oder eines der Eingangssignale eine mit Hilfe eines Sensors (1) nach einem der Ansprüche 1 bis 15 erlangte molekulargenetische Information ist.

18. Medizinisches Therapiegerät, insbesondere medizinisches Therapiegerät zur Elektrostimulation oder anderweitigen Behandlung des Herzens mit einem eingangsseitigen Sensormittel zum Erzeugen mindestens eines Eingangssignals, mit mindestens einer Verknüpfungs- und/oder Bearbeitungsstufe zum Verarbeiten des Eingangssignals zu einem Indikatorsignal, welches Indikatorsignal ein Maß für eine Therapieschwelle oder eine notwendige Therapieintensität bildet, mit mindestens einem ausgangsseitigen, von dem Indikatorsignal gesteuerten Therapie-Applikationsmittel, wobei das Eingangssignal und/oder die Eingangssignale eine mit Hilfe eines Sensors (1) nach einem der Ansprüche 1 bis 15 erlangte medizinische Information ist.

19. Medizinisches Therapiegerät, nach Anspruch 17 oder 18, welches implantierbar ausgebildet ist, wobei das Indikatorsignal, mindestens indirekt ein Eingangssignal für ein Programmiergerät bildet, welches mindestens mittelbar zur Einstellung von mindestens einem Therapie- oder Betriebszustandsparameter des Therapiegeräts im implantierten Zustand oder bereits bei der Herstellung desselben dient sowie entsprechendes Verfahren.

20. Medizinisches Therapiegerät nach einem der Ansprüche 16 bis 19, wobei mittels des mindestens einen Indikatorsignals ein Adressiersignal für einen Speicher erzeugbar ist, der nach Art einer adressierbaren Tabelle (Look-up-table) jeweils in verschiedenen von dem Adressiersignal in Abhängigkeit von dem Indikatorsignal auswählbaren Speicherbereichen als Ausgangssignale auslesbare Therapieinformationen enthält, welche dem jeweiligen Indikatorsignal zugeordnet sind.
